# EUROPEAN PATENT APPLICATION

(11) **EP 2 409 702 A1**
(43) Date of publication of application: **25.01.2012**
(21) Application number: 10007536.5
(22) Date of filing: 21.07.2010
(51) Int. Cl.: A61K 31/437, A61K 9/00, A61K 47/24

(54) **Combination of siloxane and active ingredient for treating dental disorder of hard tissues**

(71) Applicant: Bayer Innovation GmbH, 40225 Düsseldorf (DE)
(72) Inventor: Schulz, Hans H., 51067 Köln (DE)
(74) Representative: Bülle, Jan

(57) **Abstract**

The invention relates to a pharmaceutical composition comprising a siloxane according to general formula (I) 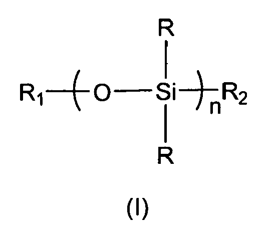 wherein
R means -C₁-C₆-alkyl;
R₁ and R₂ each mean independently of one another -C₁-C₆-alkyl, or together form a (2n)-membered ring;
and n is an integer of from 2 to 8;

for use in combination with a pharmacologically active ingredient in the prophylaxis or treatment of a dental disorder.

## Description

### FIELD OF THE INVENTION

The invention relates to a pharmaceutical composition comprising a siloxane according to general formula (I) 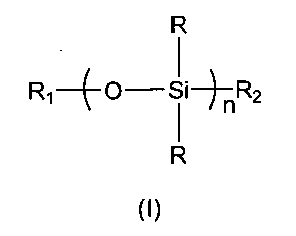 wherein
R means -C₁-C₆-alkyl;
R₁ and R₂ each mean independently of one another -C₁-C₆-alkyl, or together form a (2n)-membered ring; and
n is an integer of from 2 to 8;
for use in combination with a pharmacologically active ingredient in the prophylaxis or treatment of a dental disorder, preferably for use in restorative dentistry.

### BACKGROUND OF THE INVENTION

Siloxanes are known from the prior art and are widely used as volatile carriers in cosmetic formulations, skin care, hair care, oral care and nail care products, sunblocks, deodorants, antiperspirants, liquid bandages, lip sticks and other decorative cosmetics. The compounds have been reported to exhibit a pleasant feeling on the skin which combined with their relatively high volatility makes them particularly suitable for cosmetic formulations (cf., e. g. WO 2003/042221). WO 2007/013175 discloses a teeth whitener comprising hexamethylcyclotrisiloxane, octamethylcyclotetrasiloxane, dodecamethylcyclohexasiloxane and glycerin.

Dental disorders such as tooth decay (caries), endodontic disorders and periodontal disorders are frequently caused by bacteria.

Tooth decay (e.g., caries) is caused by microbes specific to the mouth. Oral bacteria convert dietary carbohydrates to acids capable of dissolving the tooth enamel (enamelum) and dentin (dentinum). If the enamel surface is broken, the bacteria penetrate further in the direction of the pulp into the underlying dentin and soften the dentin cone. The dentin has radial channels (dentinal tubules), running through it. It is known that the microorganisms and their toxins follow the dentinal tubules, so partial or total infection and hence inflammation of the pulp occurs as the situation develops. If the caries extensively penetrates the dentin layer or reaches the pulp, the condition is referred to as caries profunda.

The particular problems associated with treating the pulp are the small lymph supply, its position as a terminal organ with a small collateral circulation, and the influence of external stimuli given its location in a rigid, inflexible cavity.

For the treatment of caries profunda usually calcium hydroxide preparations or zinc oxide/clove oil (eugenol) are used. Both medicaments are strongly alkaline, have an antibacterial action and induce caustic necrosis in a local region of the pulp. The infected and necrotized pulp is removed as far as possible and the pulp cavity is filled with suitable root fillers (endodontic treatment). If the pulp is gangrenous, success can only be expected if the contaminated root canal system and the dentin near the canal can be disinfected. The main problems are the inaccessibility of the bacterially infected apical delta of the root canals, which is difficult to clean, and also the infected canal wall system, which has to be removed with considerable effort using instruments.

Antibiotics have been used in dental medicine both systemically and topically to treat patients. In many cases bacterial infections in the oral region can be successfully treated by the administration of systemically active antibiotics, but the systemic administration of an antibiotic necessarily burdens the entire organism. This may be contraindicated in many cases, for example due to the patient's clinical situation, if the patient has other diseases or if an allergizing potential exists. It is advantageous in such cases to apply the antibiotic topically and/or locally so that it can also be used in a higher local tissue concentration.

The main challenge facing the topical and/or local application of antibiotics in restorative dentistry is to find an antibiotic that penetrates deeply into the contaminated oral region. This is especially crucial when the root canal system and/or the surrounding dentin with the dentinal tubules are concerned. In many cases the bacterial infection has been detected late and the pathogenic microbes are already present in the lateral branches of the pulp, in the apical delta, in the periapical region and in the outer dentin layer of the pulp cavity. It is known that infected tissue cannot be completely removed with instruments in many of these canal regions because of the anatomical conditions. The diverse branches, for example an apical delta or lateral branches, and the medullary canals cannot normally be reached and prepared with instruments. The usual disinfectants are also often unsuccessful. A particularly difficult situation arises when the microbes have advanced beyond the root canal aperture (foramen apicale). This periapical region cannot be reached with root canal instruments or disinfectants in the form of root canal inserts. In this case, an antibiotic has to be applied that penetrates deeply into the periapical region and destroys all the microbes.

Another challenge facing the topical and/or local application of antibiotics in restorative dentistry is to find an antibiotic that elicits only a low tendency to generate antibiotic resistance and exhibits not only bacteriostatic action but bactericidal action. Many antibiotics only exhibit bacteriostatic action, which allows resistant microbes to get out of control and hypersensitivity reactions against a particular antibiotic can be triggered. If the antibiotic either does not reach the whole infected area or does not exhibit bactericidal action, there is always the danger of residual microbes being left behind.

EP 1 408 034 A1 discloses the use of certain derivatives of quinolonecarboxylic acid or naphthyridonecarboxylic acid and/or 4H-oxoquinolizines for the preparation of a medicament for the topical and/or local treatment and/or prophylaxis or treatment of diseases caused by bacteria in the oral region and/or tooth region and/or jaw region, for perioperative prophylaxis or for the topical and/or local treatment of diabetic foot syndrome in humans or animals. Quinolones and fluoroquinolones belong to the class of gyrase inhibitors and are thus bactericidal antibiotics.

JP 2002/179569 (A) relates to a composition for the oral cavity that is characterized by formulating a silicon compound in the composition for the oral cavity comprising a hydroxyl-2-pyridone derivative.

Problems associated with the penetration of tissue by a pharmaceutical agent have been approached by the use of so-called penetration enhancers. Volatile solvents have been used, for instance, in combination with a drug formulation in order to create a supersaturated system immediately before or during topical application. Through the resulting high concentration barrier between the formulation and the skin, the flux of the drug into the skin is increased.

WO 2007/031753 discloses a pharmaceutical formulation capable of forming a film on topical administration, said formulation comprising a preparation of a pharmaceutical, a solvent therefor, a film-forming agent, and a propellant, wherein the formulation is monophasic and the pharmaceutical is present in a substantially saturating amount therein, under conditions of use.

Other penetration enhancers, for example, increase drug partitioning into the tissue by acting as a solvent for the permeant, fluidize the lipid channels between corneocytes of the skin, disrupt the packing motif of the tissue or exert an influence on the thermodynamic activity/solubility of the drug in its vehicle, or otherwise enhance delivery into tissue. They can use a direct route through the tissue or an indirect route via channels within the tissue. Generally, penetration enhancers are drug substance- and vehicle-dependent and therefore are not universally effective.

WO 2003/043593 relates to the use of cationic surfactants as activity enhancers of the traditional antimicrobials and preparations for use in deodorants and oral care.

In the prophylaxis or treatment of dental disorder, however, the infected area that has to be reached by the antibiotic can be divided into hard tissue and soft tissue. Bacterial infections in soft tissue include infections in the pulp, in the periodontal tissue, in the gingival mucosa, in the alveolar mucosa, in the labial and buccal mucosa, in the palatine mucosa and in the periglottis. In contrast, bacterial infections in hard tissue include infections in the crown and root dentin, in the dentin inside the root canal and in the root cement in the apical region of the root of the tooth. Also included are bacterial infections of the jawbone and alveolar bone.

While many methods have been developed concerning the penetration into soft issue, the penetration into hard tissue still represents a challenge.

Dental disorder, such as tooth decay, caries, endodontic disorders and periodontic disorders, is usually caused by bacteria in the hard dental tissue. For enhancing the penetration of an antibiotic in one or more of these infected areas, a penetration enhancer has to be found that either enables the antibiotic to penetrate hard tissue directly or to proceed along the path of the bacteria through the dentinal tubules, the root canals, the diverse branches of the apical delta, the lateral branches, and/or the medullary canals.

It is an object of the invention to provide a pharmaceutical composition for the treatment of dental disorders that has advantages compared to the pharmaceutical compositions of the prior art. In particular, the pharmaceutical composition should be useful also for the treatment of bacterial infections in hard tissue such as infections in the crown and root dentin, in the dentin inside the root canal, in the root cement in the apical region of the root of the tooth, of the jawbone and alveolar bone.

This object has been achieved by the subject-matter of the patent claims.

### SUMMARY OF THE INVENTION

The invention relates to a pharmaceutical composition comprising a siloxane according to general formula (I) 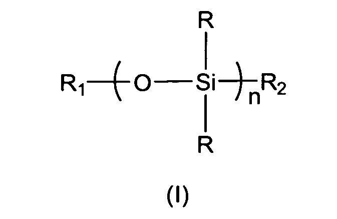 wherein
R means -C₁-C₆-alkyl;
R₁ and R₂ each mean independently of one another -C₁-C₆-alkyl, or together form a (2n)-membered ring;
and n is an integer of from 2 to 8;
for use in combination with a pharmacologically active ingredient in the prophylaxis or treatment of a dental disorder, preferably for use in restorative dentistry.

It has been surprisingly found that problems concerning the penetration of a pharmacologically active ingredient into hard dental issue can be overcome by the combined use of a pharmacologically active ingredient and a siloxane. Further, it has been surprisingly found that the siloxane acts as a penetration enhancer enabling the antibiotic to penetrate into hard tissue and to develop its therapeutic effect directly at the source of bacterial infection. It has been surprisingly found that by means of this combined administration of penetration enhancer and a pharmacologically active ingredient, the pain suffered by a patient with dental disorder can rapidly be alleviated and the number of sessions needed for the treatment of dental disorder, especially endodontic disorder, can be reduced. Moreover, there is experimental evidence that the combination of a siloxane and an antibiotic is even effective in the treatment of infected tissue, whose access is complicated by the presence of artificial tissue, e. g. gutta percha material. It appears that not only antibiotics are capable of penetrating hard tissue when combined with siloxane penetration enhancers, but that also pharmacologically active ingredients other than antibiotics can be introduced into dental hard tissue by the same pathway.

### DETAILED DESCRIPTION OF THE INVENTION

The pharmaceutical composition according to the invention comprises a siloxane according to general formula (I) 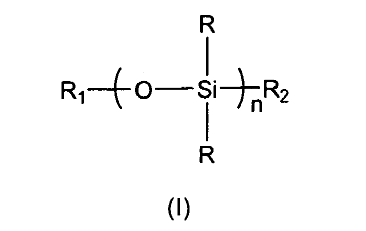 wherein
R means -C₁-C₆-alkyl, preferably C₁-C₃-alkyl, more preferably -CH₃;
R₁ and R₂ each mean independently of one another -C₁-C₆-alkyl, or together form a (2n)-membered ring;
and n is an integer of from 2 to 8.

In a preferred embodiment, the compound according to general formula (I) is a linear siloxane, i.e. R₁ and R₂ each mean independently of one another -C₁-C₆-alkyl. Preferably, R is -CH₃ or -CH₂CH₃ and/or n is an integer of from 2 to 4, preferably 2, 3 or 4.

Preferably, the linear siloxane is selected from the group consisting of hexa-C₁-C₆-alkyldisiloxanes, octa-C₁-C₆-alkyltrisiloxanes, and deca-C₁-C₆-alkyltetrasiloxanes, wherein the individual alkyl residues can be composed of same or different numbers of carbon atoms and may be independently branched or unbranched.

Preferred examples of linear hexa-C₁-C₆-alkyldisiloxanes include hexamethyldisiloxane, 1,1,3,3,3-pentamethyl-1-ethyldisiloxane, 1,1,3,3-tetramethyl-1,3-diethyldisiloxane, 1,1,3,3,3-pentamethyl-1-propyldisiloxane, 1,1,3,3-tetramethyl-1,3-dipropyldisiloxane, 1,1,3,3,3-pentamethyl-1-butyldisiloxane, and 1,1,3,3-tetramethyl-1,3-dibutyldisiloxane.

Preferred examples of linear octa-C₁-C₆-alkyltrisiloxanes include octamethyltrisiloxane, 1,1,1,3,5,5,5-heptamethyl-3-ethyl-trisiloxane, 1,1,1,5,5,5-hexamethyl-3,3-diethyl-trisiloxane, 1,1,3,3,5,5-hexamethyl-1,5-diethyl-trisiloxane, 1,1,1,3,5,5,5-heptamethyl-3-propyl-trisiloxane, 1,1,1,5,5,5-hexamethyl-3,3-dipropyl-trisiloxane and 1,1,1,3,5,5,5-heptamethyl-3-butyl-trisiloxane.

In another preferred embodiment, the compound according to general formula (I) is a cyclic siloxane according to general formula (I'), i.e. R₁ and R₂ together form a (2n)-membered ring and n is an integer of from 2 to 8:

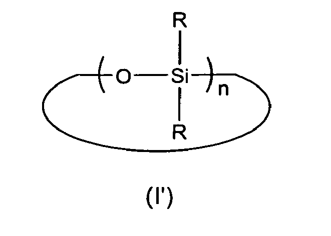

Preferably, the cyclic siloxane is selected from the group consisting of tetra-C₁-C₆-alkylcyclodisiloxanes (II), hexa-C₁-C₆-alkylcydotrisiloxanes (III), octa-C₁-C₆-alkylcyclo-tetrasiloxanes (IV), deca-C₁-C₆-alkylcyclopentasiloxanes (V) and dodeca-C₁-C₆-alkylcyclohexasiloxanes (VI), wherein the individual alkyl residues can be composed of same or different numbers of carbon atoms and may be independently branched or unbranched, i.e. compounds according to general formulas (II), (III), (IV), (V) and (VI), respectively, wherein R is independently -C₁-C₆-alkyl, preferably -methyl, -ethyl or -propyl:

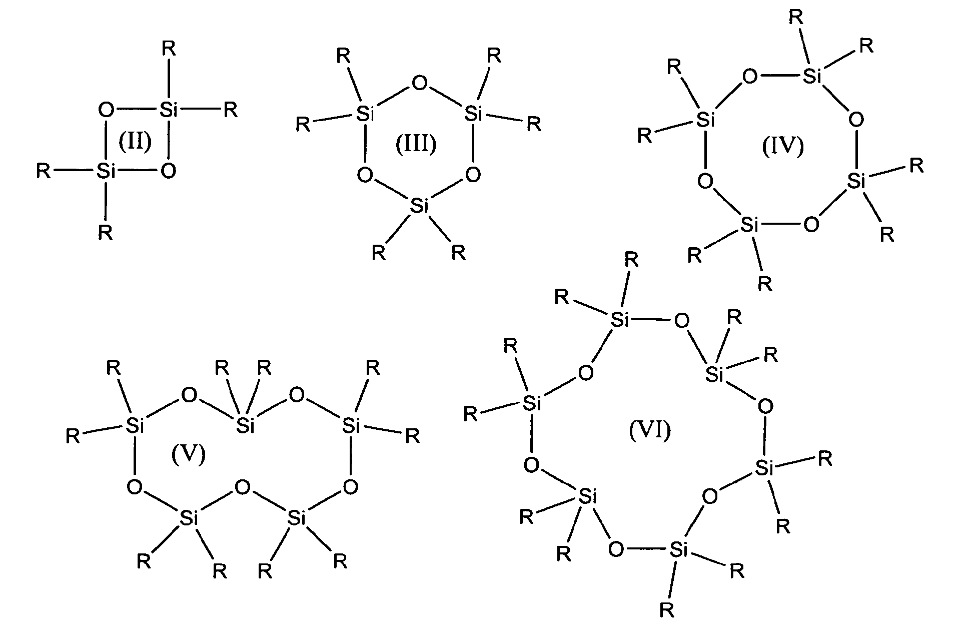

In an especially preferred embodiment, the cyclic siloxane has a structure according to general formula (I), wherein R means -CH₃, R₁ and R₂ together form a (2n)-membered ring and n is an integer of from 3 to 6, preferably 3, 4, 5 or 6; especially preferred are decamethylcyclopentasiloxane, dodecamethylcyclohexasiloxane, and, in particular, octamethylcyclotetrasiloxane.

Preferably, the siloxane according to general formula (I) has a melting point of at most 40 °C, more preferably at most 35 °C, even more preferably at most 30 °C, most preferably at most 25 °C, and in particular at most 20 °C.

Preferably, the siloxane according to general formula (I) has a boiling point at ambient pressure of at most 250 °C, more preferably at most 235 °C, even more preferably at most 220 °C, yet more preferably at most 200 °C, most preferably at most 190 °C, and in particular at most 180 °C.

In a preferred embodiment, the siloxane according to general formula (I) has a boiling point at ambient pressure of 180±60°C, more preferably 180±50°C, even more preferably 180±40°C, yet more preferably 180±30°C, most preferably 180±20°C, and in particular 180±10°C. In another preferred embodiment, the siloxane according to general formula (I) has a boiling point of 150±60°C, more preferably 150±50°C, even more preferably 150±40°C, yet more preferably 150±30°C, most preferably 150±20°C, and in particular 150±10°C. In another preferred embodiment, the siloxane according to general formula (I) has a boiling point of 120±60°C, more preferably 120±50°C, even more preferably 120±40°C, yet more preferably 120±30°C, most preferably 120±20°C, and in particular 120±10°C. In yet another preferred embodiment, the siloxane according to general formula (I) has a boiling point of 90±60°C, more preferably 90±50°C, even more preferably 90±40°C, yet more preferably 90±30°C, most preferably 90±20°C, and in particular 90±10°C.

Preferably, the siloxane according to general formula (I) has a molecular weight of at most 450 g/mol, more preferably at most 425 g/mol, even more preferably at most 400 g/mol, yet more preferably at most 375, most preferably at most 350 g/mol, and in particular at most 325 g/mol or 300 g/mol.

In a preferred embodiment, the siloxane according to general formula (I) has a molecular weight of 370±50 g/mol, more preferably 370±40 g/mol, even more preferably 370±30 g/mol, most preferably 370±20 g/mol, and in particular 370±10 g/mol. In another preferred embodiment, the siloxane according to general formula (I) has a molecular weight of 245±50 g/mol, more preferably 245±40 g/mol, even more preferably 245±30 g/mol, most preferably 245±20 g/mol, and in particular 245±10 g/mol. In yet another preferred embodiment, the siloxane according to general formula (I) has a molecular weight of 220±50 g/mol, more preferably 220±40 g/mol, even more preferably 220±30 g/mol, most preferably 220±20 g/mol, and in particular 220±10 g/mol. In another preferred embodiment, the siloxane according to general formula (I) has a molecular weight of 295±50 g/mol, more preferably 295±40 g/mol, even more preferably 295±30 g/mol, most preferably 295±20 g/mol, and in particular 295±10 g/mol.

In a preferred embodiment, the siloxane according to general formula (I) has a density of 0.96±0.10 g/mL, more preferably 0.96±0.08 g/mL, even more preferably 0.96±0.06 g/mL, yet more preferably 0.96±0.04 g/mL, most preferably 0.96±0.02 g/mL, and in particular 0.96±0.01 g/mL. In another preferred embodiment, the siloxane according to general formula (I) has a density of 0.92±0.10 g/mL, more preferably 0.92±0.08 g/mL, even more preferably 0.92±0.06 g/mL, yet more preferably 0.92±0.04 g/mL, most preferably 0.92±0.02 g/mL, and in particular 0.92±0.01 g/mL. In another preferred embodiment, the siloxane according to general formula (I) has a density of 0.88±0.10 g/mL, more preferably 0.88±0.08 g/mL, even more preferably 0.88±0.06 g/mL, yet more preferably 0.88±0.04 g/mL, most preferably 0.88±0.02 g/mL, and in particular 0.88±0.01 g/mL. In another preferred embodiment, the siloxane according to general formula (I) has a density of 0.84±0.10 g/mL, more preferably 0.84±0.08 g/mL, even more preferably 0.84±0.06 g/mL, yet more preferably 0.84±0.04 g/mL, most preferably 0.84±0.02 g/mL, and in particular 0.84±0.01 g/mL. In another preferred embodiment, the siloxane according to general formula (I) has a density of 0.80±0.10 g/mL, more preferably 0.80±0.08 g/mL, even more preferably 0.80±0.06 g/mL, yet more preferably 0.80±0.04 g/mL, most preferably 0.80±0.02 g/mL, and in particular 0.80±0.01 g/mL. In another preferred embodiment, the siloxane according to general formula (I) has a density of 0.76±0.10 g/mL, more preferably 0.76±0.08 g/mL, even more preferably 0.76±0.06 g/mL, yet more preferably 0.76±0.04 g/mL, most preferably 0.76±0.02 g/mL, and in particular 0.76±0.01 g/mL.

The pharmaceutical composition according to the invention is preferably used in combination with a pharmacologically active ingredient in the prophylaxis or treatment of a dental disorder.

In a preferred embodiment, the pharmacologically active ingredient is an antibiotic. For the purpose of specification the wording "antibiotic" herein shall include naturally occurring antibiotics as well as synthetic compounds that exhibit an antibacterial action.

Preferably, the pharmacologically active ingredient according to the invention exhibits antibiotic action and has an antibacterial spectrum against Gram-positive and Gram-negative microbes.

In a preferred embodiment, the pharmacologically active ingredient according to the invention is a bactericidal antibiotic.

The antibiotic may be selected from the group of β-lactams including penicillins such as amoxicillin, ampicillin, mezlocillin and penicillin itself, cephalosporins such as cefuroxime, cefuroxime axetil, cefadroxil, cephradine and cephalexin; aminoglycosides such as paromonycin and neomycin; and other β-lactam antibiotics such as cefachlor, ceftazidime, imipenem, meropenem, penems, carbapenems, cefpodoxime proxetil, ceftriaxone and cefoxitin. The antibiotic may further be selected from the group of tetracyclines including tetracycline itself, chlorotetracycline, oxytetracycline, demeclocyclin, doxycyclin, lymecyclin, meclocyclin, methacyclin, minocyclin, rolitetracyclin, and tigecyclin; the group of polypeptides including bacitracin, tyrothricin, colistin and polymyxin B; the group of macrolides including erythromycin and azithromycin; the group of lincosamides including clindamycin; the group of aminoglycosides including paromomycin, kanamycin, mupirocin and neomycin; the group of fluoroquinolones including ofloxacin, ciprofloxacin, moxifloxacin, pefloxacin and norfloxacin; rifamycins such as rifampicin; and nitroimidazoles such as metronidazole; and the physiologically acceptable salts thereof.

In a preferred embodiment, the pharmacologically active ingredient according to the invention is a gyrase inhibitor, in particular a fluoroquinolone, or a physiologically acceptable salt thereof.

Preferably, the fluoroquinolone is selected from the group consisting of clinafloxacin, gatifloxacin, gernifloxacin, grepafloxacin, levofloxacin, moxifloxacin, sparfloxacin, tosufloxacin trovafloxacin, and the physiologically acceptable salts thereof. Especially preferred is moxifloxacin hydrochloride.

In another preferred embodiment, the pharmacologically active ingredient according to the invention is a tetracycline such as doxycyclin and minocyclin, or a physiologically acceptable salt thereof.

Preferably, the antibiotic is selected from the group consisting of doxycyclin, minocyclin, moxifloxacin, and the physiologically acceptable salts thereof.

The pharmacologically active ingredient may be present as an acid addition salt, whereby any suitable acid capable of forming such an addition salt may be used. Suitable acids include but are not limited to hydrochloric acid, hydrobromic acid, methanesulphonic acid and toluenesulphonic acid. If the pharmacologically active ingredient contains one or more acidic groups e. g. carboxylic groups, it may be present in its neutralized form, preferably as an alkali metal salt, alkaline earth metal salt, ammonium salt, guanidinium salt or silver salt.

According to the invention, the pharmaceutical composition comprising the siloxane according to general formula (I), preferably octamethylcyclotetrasiloxane, is intended for being used in combination with the pharmacologically active ingredient.

Preferably, the administration of the siloxane, preferably octamethylcyclotetrasiloxane, and/or of the pharmacologically active ingredient proceeds topically and/or locally.

In a preferred embodiment, the siloxane according to general formula (I) , preferably octamethylcyclotetrasiloxane, and the pharmacologically active ingredient are administered in a relative molar ratio of > 1:1, more preferably > 2:1, still more preferably > 4:1, yet more preferably > 6:1, even more preferably > 8:1, most preferably > 10:1, and in particular > 15:1.

The active ingredient may either be contained in the pharmaceutical composition comprising the siloxane, preferably octamethylcyclotetrasiloxane, (for the purpose of the specification also referred to as "combined pharmaceutical composition") or in a separate pharmaceutical composition (for the purpose of the specification also referred to as "second pharmaceutical composition").

Unless expressly stated otherwise, the general term "pharmaceutical composition" refers to both embodiments, i.e. the combined pharmaceutical combination as well as the first pharmaceutical composition and the second pharmaceutical composition, respectively.

Preferably, administration of the combined pharmaceutical composition and the first and/or second pharmaceutical composition, respectively, proceeds topically and/or locally.

In a preferred embodiment, the pharmaceutical composition according to the invention comprises both the pharmacologically active ingredient and the siloxane according to general formula (I), preferably octamethylcyclotetrasiloxane (in the following also referred to as "combined pharmaceutical composition").

Preferably, the content of the siloxane according to general formula (I) , preferably octamethylcyclotetrasiloxane, in the combined pharmaceutical composition is at least 0.1 wt.-%, more preferably at least 1 wt.-%, still more preferably at least 5 wt.-%, yet more preferably at least 10 wt.-%, even more preferably at least 25 wt.-%, most preferably at least 50 wt.-% and in particular at least 75 wt.-%, based on the total weight of the combined pharmaceutical composition.

Preferably, the content of the pharmacologically active ingredient in the combined pharmaceutical composition is at most 50 wt.-%, more preferably at most 40 wt.-%, still more preferably at most 30 wt.-%, yet more preferably at most 20 wt.-%, even more preferably at most 15 wt.-%, most preferably at most 10 wt.% and in particular at most 5 wt.-%, based on the total weight of the combined pharmaceutical composition.

In a preferred embodiment, the concentration of the pharmacologically active ingredient in the combined pharmaceutical composition is within the range of from 0.01 mg/mL to 200 mg/mL, more preferably from 0.1 mg/mL to 150 mg/mL, even more preferably from 1 mg/mL to 100 mg/mL, yet more preferably at most from 2 mg/mL to 80 mg/mL, most preferably from 3 mg/mL to 60 mg/mL, and in particular from 5 mg/mL to 40 mg/mL.

In another preferred embodiment, the concentration of the pharmacologically active ingredient in the combined pharmaceutical composition is at most 100 mg/mL, more preferably at most 75 mg/mL, even more preferably at most 50 mg/mL, yet more preferably at most 40 mg/mL, most preferably at most 30 mg/mL and in particular at most 20 mg/mL.

In a preferred embodiment, the relative molar ratio of the siloxane according to general formula (I), preferably octamethylcyclotetrasiloxane, and the pharmacologically active ingredient in the combined pharmaceutical composition, is preferably > 1:1, more preferably > 2:1, still more preferably > 4:1, yet more preferably > 6:1, even more preferably > 8:1, most preferably > 10:1, and in particular > 15:1.

In a preferred embodiment, the combined pharmaceutical formulation is a liquid. In another preferred embodiment, the combined pharmaceutical formulation has a gelatinous consistency.

In a preferred embodiment, the administration of the combined pharmaceutical composition proceeds topically and/or locally.

A further aspect of the invention relates to the combined pharmaceutical composition as described above and herein below.

In another preferred embodiment, the siloxane according to general formula (I), preferably octamethylcyclotetrasiloxane, is contained in the pharmaceutical composition (in the following also referred to as "first pharmaceutical composition"), whereas the pharmacologically active ingredient is contained in as a second, separate pharmaceutical composition.

Preferably, the content of the siloxane according to general formula (I), preferably octamethylcyclotetrasiloxane, in the first pharmaceutical composition is at least 75 wt.-%, more preferably at least 80 wt.-%, still more preferably at least 85 wt.-%, yet more preferably at least 90 wt.-%, even more preferably at least 95 wt.-%, most preferably at least 97 wt.% and in particular 98 wt.-%, based on the total weight of the first pharmaceutical composition.

Preferably, the content of the pharmacologically active ingredient in the second pharmaceutical composition is at most 50 wt.-%, more preferably at most 40 wt.-%, still more preferably at most 30 wt.-%, yet more preferably at most 20 wt.-%, even more preferably at most 15 wt.-%, most preferably at most 10 wt.-% and in particular at most 5 wt.-%, based on the total weight of the combined pharmaceutical composition.

Preferably, the concentration of the pharmacologically active ingredient in the second pharmaceutical composition is within the range of from 0.01 mg/mL to 200 mg/mL, more preferably from 0.1 mg/mL to 150 mg/mL, even more preferably from 1 mg/mL to 120 mg/mL, yet more preferably at most from 5 mg/mL to 100 mg/mL, most preferably from 10 mg/mL to 80 mg/mL, and in particular from 15 mg/mL to 60 mg/mL.

Preferably, the concentration of the pharmacologically active ingredient in the second pharmaceutical composition is within the range of 50±45 mg/mL, more preferably 50±40 mg/mL, even more preferably 50±30 mg/mL, yet more preferably 50±20 mg/mL, most preferably 50±10 mg/mL, and in particular 50±5 mg/mL. In a preferred embodiment, the concentration of the pharmacologically active ingredient in the second pharmaceutical composition is within the range of 30±25 mg/mL, more preferably 30±20 mg/mL, even more preferably 30±15 mg/mL, yet more preferably 30±10 mg/mL, and most preferably 30±5 mg/mL. In another preferred embodiment, the concentration of the pharmacologically active ingredient in the second pharmaceutical composition preferably is within the range of 20±15 mg/mL, more preferably 20±10 mg/mL, and most preferably 20±5 mg/mL. In yet another preferred embodiment, the concentration of the pharmacologically active ingredient in the second pharmaceutical composition preferably is within the range of 15±10 mg/mL, more preferably 15±7.5 mg/mL, and most preferably 15±5 mg/mL.

The first pharmaceutical composition according to the invention may be administered prior to, simultaneously with and/or subsequently to the second pharmaceutical composition according to the invention. It is also possible to divide the first and the second pharmaceutical composition in subunits and to administer some subunits prior to, simultaneously with and/or subsequently to other subunits. For example, the first pharmaceutical composition may be divided into two subunits and the first subunit is administered in the beginning. Thereafter, the second pharmaceutical composition is administered. The second subunit of the first pharmaceutical composition may then either be administered simultaneously with or subsequently to the second pharmaceutical composition.

The present invention also encompasses administration regimens that combine the administration of a combined pharmaceutical composition according to the invention prior to, simultaneously with and/or subsequently to administration of a first and/or second pharmaceutical composition according to the invention, respectively.

Independent of the order of administration, the length of the period between administration of both, i.e. first and second pharmaceutical compositions is preferably at most 30 minutes, more preferably at most 20 minutes, even more preferably at most 15 minutes, yet more preferably at most 10 minutes, still more preferably at most 5 minutes, most preferably at most 3 minutes, and in particular at most 2 minutes.

Preferably, the first pharmaceutical composition (comprising the siloxane, preferably octamethylcyclotetrasiloxane) is administered prior to and/or simultaneously with at least part of the second pharmaceutical composition (comprising the pharmacologically active ingredient). Another part of the second pharmaceutical composition may be administered before the first pharmaceutical composition or at any other time during the treatment.

In a preferred embodiment, the first pharmaceutical composition comprising the siloxane according to general formula (I), preferably octamethylcyclotetrasiloxane, and the second, separate pharmaceutical composition comprising a pharmacologically active ingredient are contained in a kit.

In a preferred embodiment, the first pharmaceutical composition and the second pharmaceutical composition are both liquids.

In another preferred embodiment, the first pharmaceutical composition and the second pharmaceutical composition are both gelatinous formulations.

In yet another pharmaceutical composition, the first pharmaceutical composition is a gelatinous formulation and the second pharmaceutical composition is a liquid.

In an especially preferred embodiment, the first pharmaceutical composition is a liquid and the second pharmaceutical composition is a gelatinous formulation.

The second pharmaceutical composition containing the pharmacologically active ingredient, is preferably present in form of a solution, gel, suspension, emulsion, liposomes or micelles.

A further aspect of the invention relates to a kit comprising the first pharmaceutical composition as described above or hereinafter and the second pharmaceutical composition as described above or hereinafter in formulation spatially separated from one another.

In a preferred embodiment, the administration of the first pharmaceutical composition and the second pharmaceutical composition proceeds topically and/or locally.

The pharmaceutical composition according to the invention, i.e. the combined pharmaceutical composition and the first and/or second pharmaceutical composition, respectively, is/are preferably independent of one another a solution, gel, suspension, emulsion, in form of liposomes or micelles.

Examples of solutions are aqueous solutions in the presence of solubilizers.

Examples of solubilizers are salts, polyols, sugar alcohols, polyglycols or cosolvents such as glycerol, ethylene glycol, propylene glycol, furfural, N,N-dimethylformamide, methanol, ethanol, i-propanol, n-propanol or acetone.

Aqueous gels can be prepared by the addition of gelling agents such as pectins, ethylene glycol monomethacrylate gel, alginates, hydroxyethyl cellulose, carboxymethyl hydroxyethyl cellulose, polyglyceryl methacrylates or polysaccharides. Other suitable additives are thickeners such as cellulose, alkyl cellulose, hydroxyethyl cellulose, agar-agar, carboxymethyl guar and cellulose ethers, or hydrotropic solubilizers such as ethylenediamine, urea or cyclodextrins.

The galenical forms can also contain solubilizers, such as surfactants, or preservatives. Examples of possible suspension constituents are tragacanth, cellulose, wetting agents, glycols, polyols, mucins or cellulose ethers. Possible emulsion constituents are emulsifiers such as polysorbates, surfactants, lecithins, mucins, gelatin or carboxymethyl cellulose.

Other suitable forms of administration include dental pocket inserts comprising an inert carrier material, which are impregnated with the pharmacologically active ingredient and/or the siloxane according to general formula (I) , preferably octamethylcyclotetrasiloxane, and optionally other auxiliary substances and gradually release the active ingredient by dissolution. Examples of possible forms of administration for root fillings are tampons, cotton wool plugs or foam pellets. Application in the case of soft tissue infections can be effected with strips or thread inserts. It is also possible to use pharmaceutical substances or auxiliary substances for osmotic adjustment. Other possible auxiliary substances for formulating the pharmacologically active ingredient are antioxidants, chelating agents, disinfectants, dispersants, emulsion stabilizers, hydrocolloids, preservatives, solubilizers, wetting agents, quaternary ammonium compounds, stabilizers, suspending agents or thickeners. The above-mentioned constituents can also be used in combination with one another.

Suitable stable gelatinous formulations are those which, apart from the pharmacologically active ingredient, are composed of polyethers, modified celluloses and water. Preferred gel formulations are those containing propylene glycol, Tween 20 solution and muc. hydroxyethyl cellulose.

Especially preferred compositions contain the pharmacologically active ingredient in amounts of 0.001 to 100 mg/ml and/or the siloxane according to general formula (I), preferably octamethylcyclotetrasiloxane, in amounts of 100 to 950 mg/mL, polypropylene glycol in amounts of 5 to 250 mg/ml, 1% Tween 20 solution in amounts of 5 to 200 mg/ml and muc. hydroxyethyl cellulose ad 1 g/ml.

Particularly preferred gelatinous formulations contain the pharmacologically active ingredient in amounts of 1 to 100 mg/ml, and/or the siloxane according to general formula (I), preferably octamethylcyclotetrasiloxane, in amounts of 200 to 950 mg/mL and/or propylene glycol in amounts of 50 to 200 mg/ml, 1% Tween 20 solution in amounts of 3 to 150 mg/ml and muc. hydroxy cellulose ad 1 g/ml.

In a preferred embodiment, the combined pharmaceutical combination and the first and/or second pharmaceutical composition, respectively, are provided as individual dosage units, which are administered as such.

In another preferred embodiment, the combined pharmaceutical combination and the first and/or second pharmaceutical composition, respectively, are provided as multiple dosage form, from which the individual dosage units are separated and administered. The individual dosage units may preferably be separated from the multiple dosage unit form by means of a pipette or syringe. A typical example for a multiple dosage form according to the invention is an optionally sterilized glass container sealed with a septum. Said glass container contains a volume of the pharmaceutical combination well exceeding the individual volume of an individual dosage unit that is intended for at once administration to the patient. For example, when the glass container contains a volume of 10 ml and the individual dosage unit that is intended for at once administration to the patient amounts to, e.g., 0.2 ml, the glass container is a multiple dosage form suitable for repeated use fifty times.

In a preferred embodiment, the multiple dosage unit form is a gelatinous formulation or a liquid, which is contained preferably in a container, in particular a glass container.

Preferably, the multiple dosage unit form contains at least 2, more preferably at least 5, even more preferably at least 10, yet more preferably at least 15, most preferably at least 20, and in particular 25 individual dosage units.

In a preferred embodiment, the individual dosage units have a volume of 0.3±0.25 mL, more preferably of 0.3±0.2 mL, still more preferably 0.3±0.15 mL, yet more preferably of 0.3±0.1 mL, even more preferably of 0.3±0.075 mL, most preferably of 0.3±0.05 mL, and in particular of 0.3±0.025 mL.

In another preferred embodiment, the individual dosage units have a volume of 0.2±0.175 mL, more preferably of 0.2±0.15 mL, still more preferably 0.2±0.125 mL, yet more preferably of 0.2±0.1 mL, even more preferably of 0.2±0.075 mL, most preferably of 0.2±0.05 mL, and in particular of 0.2±0.025 mL.

In yet another preferred embodiment, the individual dosage units have a volume of 0.1 ±0.09 mL, more preferably of 0.1±0.08 mL, still more preferably 0.1±0.07 mL, yet more preferably of 0.1±0.06 mL, even more preferably of 0.1±0.05 mL, most preferably of 0.1±0.04 mL, and in particular of 0.1 ±0.02 mL.

The amount of the siloxane according to general formula (I), preferably octamethylcyclotetrasiloxane, that is contained in the individual dosage unit is at least 0.001 mL, more preferably at least 0.005 mL, even more preferably at least 0.01 mL, yet more preferably at least 0.025 mL, even more preferably at least 0.05 mL, most preferably at least 0.075 mL and in particular at least 0.1 mL. In a preferred embodiment, the amount of the siloxane according to general formula (I), preferably octamethylcyclotetrasiloxane, that is contained in the individual dosage unit is 0.2±0.175 mL, more preferably 0.2±0.15 mL, even more preferably 0.2±0.1 mL, yet more preferably 0.2±0.075 mL, most preferably 0.2±0.05 mL, and in particular 0.2±0.025 mL. In another preferred embodiment, the amount of the siloxane according to general formula (I), preferably octamethylcyclotetrasiloxane, that is contained in the individual dosage unit is 0.25±0.2 mL, more preferably 0.25±0.175 mL, even more preferably 0.25±0.15 mL, yet more preferably 0.25±0.1 mL, most preferably 0.25±0.075 mL, and in particular 0.25±0.05 mL. In yet another preferred embodiment, the amount of the siloxane according to general formula (I), preferably octamethylcyclotetrasiloxane, that is contained in the individual dosage unit is 0.3±0.25 mL, more preferably 0.3±0.20 mL, even more preferably 0.3±0.15 mL, yet more preferably 0.3±0.1 mL, and most preferably 0.3±0.05 mL.

According to the invention, the pharmacologically active ingredient is administered in combination with the pharmaceutical composition in a therapeutically effective amount. The amount that constitutes a therapeutically effective amount varies according to the nature of the compound, the condition being treated, the severity of said condition and the patient being treated.

Preferably, the dose of the pharmacologically active ingredient is in the range of 0.01 mg to 100 mg, more preferably in the range of 0.1 mg to 50 mg, still more preferably in the range of 0.25 mg to 40 mg, yet more preferably in the range of 0.5 mg to 30 mg, most preferably in the range of 1 mg to 20 mg, and in particular in the range of 2 mg to 15 mg.

In a preferred embodiment, the dose of the pharmacologically active ingredient preferably is within the range of 10±9 mg, more preferably 10±8 mg, still more preferably 10±7 mg, yet more preferably 10±6 mg, most preferably 10±4 mg, and in particular 10±2 mg.

In another preferred embodiment, the dose of the pharmacologically active ingredient preferably is within the range of 6±5 mg, more preferably 6±4 mg, still more preferably 6±3 mg, yet more preferably 6±2 mg, most preferably 6±1.5 mg, and in particular 6±1 mg.

In still another preferred embodiment, the dose of the pharmacologically active ingredient preferably is within the range of 4±3.5 mg, more preferably 4±3 mg, still more preferably 4±2.5 mg, yet more preferably 4±2 mg, most preferably 4±1.5 mg, and in particular 4±1 mg.

A further aspect of the invention relates to a pharmaceutical dosage form comprising a siloxane according to general formula (I), preferably octamethylcyclotetrasiloxane, as defined above or hereinafter in combination with a pharmacologically active ingredient as described above or hereinafter. Preferably, the pharmaceutical dosage form according to the invention comprises the combined pharmaceutical composition according to the invention.

The pharmaceutical composition according to the invention is for the prophylaxis or treatment of a dental disorder, preferably in restorative dentistry. The active ingredient may either be contained in the pharmaceutical composition comprising the siloxane, preferably octamethylcyclotetrasiloxane, (combined pharmaceutical composition) or in a second pharmaceutical composition.

Preferably, the dental disorder is selected from diseases caused by bacteria in the oral region of humans and animals, especially pulpitis, including infections of the root canal and the periapical tissue, periodontal diseases and odontogenous or oral soft tissue infections and dentin wounds.

In an especially preferred embodiment, the dental disorder is selected from the group consisting of tooth decay, caries, endodontic disorders and periodontal disorders.

In an especially preferred embodiment, the pharmacologically active ingredient according to the invention is an antibiotic that exhibits a beneficial action in the prophylaxis or treatment of diseases caused by bacteria in the oral region of humans and animals, especially in the prophylaxis or treatment of pulpitis, including infections of the root canal and the periapical tissue, periodontal diseases and odontogenous or oral soft tissue infections and in the prophylaxis or treatment of dentin wounds.

In an especially preferred embodiment, the dental disorder is an endodontic disease, i.e. the pharmaceutical composition comprising the siloxane according to general formula (I), preferably octamethylcyclotetrasiloxane, and the pharmacologically active ingredient are for use in endodontic treatment.

In a preferred embodiment, the pharmaceutical composition comprising the siloxane according to general formula (I), preferably octamethylcyclotetrasiloxane, and/or the pharmacologically active ingredient are for use in combination with other anti-infective agents such as antibacterial, antifungal or antiviral substances. Said other anti-infective agents may be contained in the pharmaceutical composition according to the invention or be provided in form of separate pharmaceutical compositions.

In a preferred embodiment, the pharmaceutical composition according to the invention is for use in combination with a root canal irrigant. The root canal irrigant is preferably selected from hydrogen peroxide, sodium hypochlorite, chlorhexidine gluconate, cetrimide, ethylenediaminetetraacetic acid, framycetin sulfate, doxycyclin, minocyclin, moxifloxacin or any other antibiotic.

Preferably, the endodontic treatment is conducted comprising the following steps:
a) the infected pulp is removed mechanically, e. g. by means of a nerve needle; and/or
b) the canal lumen is widened and the infected canal wall is removed; and/or
c) the root canals are flushed with a solution comprising the root canal irrigant; and/or
d) the root canals are treated with a first pharmaceutical composition comprising the siloxane according to general formula (I), preferably octamethylcyclotetrasiloxane, particularly by injection; and/or
e) the root canals are treated with a second pharmaceutical composition containing the pharmacologically active ingredient; and/or
f) the root canals are occluded in the crown region, first with a cotton wool plug and then with zinc phosphate cement to seal the margins.

Alternatively, steps d) and e) can be conducted as one step using a pharmaceutical composition containing both the siloxane according to general formula (I), preferably octamethylcyclotetrasiloxane, and the pharmacologically active ingredient. After this procedure the patient is released for preferably 4-6 days. In case that the tooth is tested odorless and insensitive towards percussion, the root canals are definitively filled, preferably with endomethasone and gutta percha. Otherwise steps c), d), e) and/or f) have to be repeated.

In another preferred embodiment, the dental disorder is a dentin wound, e.g. a disease occurred after the preparation of dentin surfaces to receive e.g. fillings, inlays, onlays, crowns or bridges. Preferably, the combination according to the invention is applied to form a local depot on the dentin surface with an antibiotic action before the reliner is placed on and/or in the fundament of the inlays, onlays, crowns or bridges. This preferably results in the effective destruction of the residual pathogens not only on the surface of the cavity, but also in the dentinal tubules.

In another preferred embodiment, the dental disorder is a periodontal disease. For this application, threads or so-called chips impregnated with solutions or gelatinous preparations of the pharmacologically active ingredient are preferably inserted in the gingival pocket. Alternatively, the pharmacologically active ingredient can be placed in a medicament carrier covering the teeth and the gingiva. Prior to, during or after the application of the pharmacologically active ingredient, the pharmaceutical composition comprising the siloxane according to general formula (I) is introduced into the gingival pocket by means of one of the two aforementioned methods. Alternatively, the pharmacologically active ingredient and/or pharmaceutical composition comprising the siloxane according to general formula (I), preferably octamethylcyclotetrasiloxane, are introduced into the gingival pockets by intraligamental injection.

In yet another preferred embodiment, the combination according to the invention is for use in the treatment of dental disorder that affects a tooth with existing root filling (secondary root treatment), in particular when attempts to remove the existing root filling at least partially fail.

Preferably, the combination according to the invention improves tissue penetration of the pharmacologically active ingredient. Preferably, the siloxane according to general formula (I), preferably octamethylcyclotetrasiloxane, promotes the transport of the pharmacologically active ingredient to the infected tissue, e. g. through dentinal tubules, lateral branches of the pulp, branches of the apical delta or the medullary canals. In a preferred embodiment, the administration of the pharmaceutical composition comprising the siloxane according to general formula (I), preferably octamethylcyclotetrasiloxane, improves penetration of the pharmacologically active ingredient into soft tissue and/or hard tissue.

In another preferred embodiment, the administration of the pharmaceutical composition comprising the siloxane according to general formula (I), preferably octamethylcyclotetrasiloxane, improves penetration of the pharmacologically active ingredient into root canals that at least partially contain a filling material, e. g. gutta percha.

In a preferred embodiment, the combination according to the invention is for use in the local and/or topical treatment of diseases caused by bacteria in hard tissue, including infections in the crown and root dentin, in the dentin inside the root canal and in the root cement in the apical region of the root of the tooth.

In a preferred embodiment, the combination according to the invention is for use in the local and/or topical treatment of bacterial infections in soft tissue, including infections in the pulp, in the periodontal tissue, in the gingival mucosa, in the alveolar mucosa, in the labial and buccal mucosa, in the palatine mucosa and in the periglottis.

In a preferred embodiment, the number of treatments with the pharmacologically active ingredient can be reduced when it is used in combination with the pharmaceutical composition comprising the siloxane according to general formula (I), preferably octamethylcyclotetrasiloxane. Preferably, the patient is free of complaints after one or two sessions during which the combination of the pharmaceutical composition comprising the siloxane according to general formula (I), preferably octamethylcyclotetrasiloxane, and the pharmacologically active ingredient is applied.

### In an especially preferred embodiment,

- the siloxane according to general formula (I) is selected from octamethylcyclotetrasiloxane, decamethylcyclopentasiloxane and decamethylcyclohexasiloxane; and/or
- the siloxane according to general formula (I) has a boiling point of at most 250 °C;
   and/or
- the content of the siloxane according to general formula (I), preferably octamethylcyclotetrasiloxane, in the pharmaceutical composition is 0.2±0.175 mL; and/or
- the pharmacologically active ingredient is an antibiotic selected from the group consisting of fluoroquinolones and tetracyclines; and/or
- the dose of the pharmacologically active ingredient is within the range of from 0.1 mg to 50 mg; and/or
- concentration of the pharmacologically active ingredient is within the range of from 0.1 mg/mL to 150 mg/mL; and/or
- the pharmacologically active ingredient is administered in form of an aqueous solution or gelatinous formulation; and/or
- the administration of the pharmaceutical composition and the pharmacologically active ingredient proceeds topically and/or locally; and/or
- the composition either comprises the pharmacologically active ingredient in combination with the siloxane according to general formula (I), preferably octamethylcyclotetrasiloxane, or the pharmacologically active ingredient is contained in as a second, separate pharmaceutical composition; and/or
- administration of the composition improves penetration of the pharmacologically active ingredient into hard tissue.

### EXAMPLES

The invention is further illustrated by the following examples which are not to be construed as limiting its scope.

### Example 1:

In a first trial, a combination of moxifloxacin and octamethylcyclotetrasiloxane was applied to gangrenous pulp of 6 randomly chosen patients (inventive examples I-1 to I-6) during endodontic treatment (root canal treatment). The procedure was conducted as follows:
- the root canals were treated mechanically;
- during and after the mechanical treatment the root canals were flushed with an Avalox®-infusion solution (commercially available Moxifloxacin solution) by means of an endo-pipette, the excess of which was removed with a paper point;
- the root canals were flushed with octamethylcyclotetrasiloxane (98%, approx. 0.25 mL) by means of an endopipette and the excess was removed with a paper point; and
- moxifloxacin hydrochloride gel (20 mg/mL moxifloxacin; approx. 0.25 mL) was introduced into the root canals by means of a lentulo spiral.

At the end of this procedure, the roots were filled temporarily and the patient was released for at least five days. During the next session the treatment with octamethylcyclotetrasiloxane and moxifloxacin was repeated according to the previous session. In case that the tooth was tested odorless and insensitive towards percussion, the root was filled permanently with endomethasone and gutta percha material. However, in case that the percussion and/or the odor test failed, the treatment with octamethylcyclotetrasiloxane and moxifloxacin was repeated during one or more additional sessions until both criteria were met.

Accordingly, a comparative study was conducted in which the same root treatment therapy was applied to gangrenous pulp of 4 randomly chosen patients with the following exception: In the first session moxifloxacin was applied without using any octamethylcyclotetrasiloxane. During the following sessions, both moxifloxacin and octamethylcyclotetrasiloxane were applied according to the first study.

The results of the first study (inventive examples I-1 to I-6) and the second study (inventive examples C-7 to C-10) are summarized in the table here at most:

| | I- 1 | I- 2 | I- 3 | I- 4 | I- 5 | I-6 | C- 1 | C- 2 | C- 3 | C-4 |
|---|---|---|---|---|---|---|---|---|---|---|
| Position of the infected tooth | 45 | 18 | 13 | 37 | 44 | 25 | 16 | 11 | 46 | 36 |
| First session | | | | | | | | | | |
| Application of octamethylcyclotetrasiloxane | + | + | + | + | + | + | - | - | - | - |
| Percussion sensitivity test | - | - | - | + | + | + | - | + | + | + |
| Second session | | | | | | | | | | |
| Application of octamethylcyclotetrasiloxane | + | + | + | + | + | + | + | + | + | + |
| Percussion sensitivity test | - | - | - | - | + | + | - | + | + | + |
| Third session | | | | | | | | | | |
| Application of octamethylcyclotetrasiloxane | - | - | - | - | + | - | - | + | + | + |
| Percussion sensitivity test | - | - | - | - | - | - | - | - | - | + |
| Fourth session | | | | | | | | | | |
| Application of octamethylcyclotetrasiloxane | - | - | - | - | + | - | - | - | - | + |
| Percussion sensitivity test | - | - | - | - | - | - | - | - | - | - |
| Free of complaints in session | 2 | 2 | 2 | 2 | 3 | 3 | 2 | 3 | 3 | 4 |

The above results demonstrate that in those cases in which the combination of moxifloxacin and octamethylcyclotetrasiloxane was directly applied in the first session, most of the patients were free of complaints after only one session and the root canal could be filled in the second session (see inventive examples I-1, I-2, I-3 and I-4). The rest of the patients were free of complaints after two treatments with moxifloxacin and octamethylcyclotetrasiloxane (I-5 and I-6).

In all other cases, in which moxifloxacin was applied without octamethylcyclotetrasiloxane in the first session and the combination of both was first employed in the second session, one of the patients were free of complaints after one sessions (C-1), two patients were free of complaints after two sessions (C-2 and C-3), and one after three session (C-4).

A comparison of the inventive examples (I-1 to I-6) to the comparative (C-1 to C-2) examples shows, that the mean length of the root treatment therapy is shortened through the combined application of moxifloxacin and octamethylcyclotetrasiloxane by one session, and most of the patients are free of complaints after a single treatment with said combination.

### Example 2:

### Secondary root treatment after failed root treatment:

Patient reported bite discomfort in tooth 15 which had been increasing steadily during the month before.

Clinical diagnosis:
Tooth 15 had been undergone canal root treatment 8 years ago and filled with gutta percha afterwards. However, the canal filling did not extend deeply enough into the canal and approx. 2 mm of the root ends (apex) remained thus unfilled. X-rays taken from the root canal did not reveal any complications.

Course of treatment:
The secondary endodontic treatment began with trepanation of the affected tooth. Attempts to remove the hardened gutta percha material completely by means of a Tri Auto ZX tool (Morita) and titanium files were only partially successful, as revealed by X-ray. A three-day canal insert with moxifloxacin hydrochloride gel (50 mg/ml) did not relieve the bite discomfort. Another canal insert with moxifloxacin hydrochloride gel (50 mg/ml) was introduced, however, this time after a pre-treatment with octamethylcyclotetrasiloxane (98%, approx. 0.25 mL). The patient reported to be free of complaints after 3 days.

## Claims

1. Pharmaceutical composition comprising a siloxane according to general formula (I) 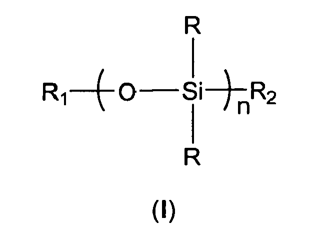 wherein
R means -C₁-C₆-alkyl;
R₁ and R₂ each mean independently of one another -C₁-C₆-alkyl, or together form a (2n)-membered ring;
and n is an integer of from 2 to 8;
for use in combination with a pharmacologically active ingredient in the prophylaxis or treatment of a dental disorder.

2. Composition according to claim 1, wherein R means -CH₃, R₁ and R₂ together form a (2n)-membered ring and n is an integer of from 3 to 6.

3. Composition according to claim 1 or 2, wherein the siloxane is octamethylcyclotetrasiloxane.

4. Composition according to any of the preceding claims, wherein the composition is administered prior to, simultaneous with and/or subsequent to the pharmacologically active ingredient.

5. Composition according to any of the preceding claims, wherein the composition either comprises the pharmacologically active ingredient in combination with the siloxane according to general formula (I), or the pharmacologically active ingredient is contained in as a second, separate pharmaceutical composition.

6. Composition according to any of the preceding claims, wherein administration of the composition and/or of the pharmacologically active ingredient proceeds topically and/or locally.

7. Composition according to any of the preceding claims, wherein administration of the composition improves penetration of the pharmacologically active ingredient into hard tissue.

8. Composition according to any of the preceding claims, wherein the pharmacologically active ingredient is an antibiotic.

9. Composition according to claim 8, wherein the antibiotic is a gyrase inhibitor.

10. Composition according to claim 8 or 9, wherein the antibiotic is a fluoroquinolone.

11. Composition according to claim 8, wherein the antibiotic is selected from the group consisting of doxycyclin, minocyclin, moxifloxacin and the physiologically acceptable salts thereof.

12. Composition according to any of the preceding claims, wherein the relative molar ratio of siloxane according to general formula (I) and pharmacologically active ingredient is > 1:1.

13. Composition according to any of the preceding claims, wherein the dental disorder is selected from the group consisting of tooth decay, caries, endodontic disorders and periodontic disorders.

14. Kit comprising a first pharmaceutical composition which comprises a siloxane according to general formula (I) as defined in any of claims 1 to 3, and a second, separate pharmaceutical composition comprising a pharmacologically active ingredient as defined in any of claims 1 or 8 to 12.

15. Kit according to claim 14, wherein the second pharmaceutical composition is a gel.

16. Pharmaceutical dosage form comprising a siloxane according to general formula (I) as defined in any of claims 1 to 3 in combination with a pharmacologically active ingredient as defined in any of claims 1 or 8 to 12.
